# EUROPÄISCHE PATENTSCHRIFT

(11) **EP 0 984 730 B1**
(45) Veröffentlichungstag und Bekanntmachung des Hinweises auf die Patenterteilung: **30.07.2003**
(21) Anmeldenummer: 97924848.1
(22) Anmeldetag: 16.06.1997
(51) Int. Cl.: A61B 17/70

(54) **VORRICHTUNG ZUR VERBINDUNG EINES LÄNGSTRÄGERS MIT EINER PEDIKELSCHRAUBE**
DEVICE FOR CONNECTING A LONGITUDINAL SUPPORT WITH A PEDICLE SCREW
DISPOSITIF POUR RELIER UN SUPPORT LONGITUDINAL AVEC UNE VIS PEDONCULAIRE

(30) Priorität: 17.05.1997 DE 19720782
(43) Veröffentlichungstag der Anmeldung: 15.03.2000
(73) Patentinhaber: SYNTHES AG CHUR, 7002 Chur (CH)
(72) Erfinder: SCHLÄPFER, Fridolin, CH-8750 Glarus (CH); HESS, Martin, CH-4434 Hölstein (CH)
(74) Vertreter: Lusuardi, Werther Giovanni, Dr.
(86) Internationale Anmeldenummer: CH9700236
(87) Internationale Veröffentlichungsnummer: WO98052482

(56) Entgegenhaltungen:
- EP-A- 0 441 729
- WO-A-97/02786
- DE-C- 19 509 332

## Beschreibung

Die Erfindung bezieht sich auf eine Vorrichtung gemäss dem Oberbegriff des Patentanspruchs 1.

Aus dem Stand der Technik sind Pedikelschrauben für die Wirbelsäulenfixation bekannt, welche den Vorteil haben, dass der Längsträger nachträglich von oben eingebracht werden kann und der Winkel zwischen Pedikelschraube und Längsträger variabel einstellbar ist. Solche Pedikelschrauben sind unter anderem aus den Patenten EP-B 0 330 881 SHERMAN und EP-B 0 441 729 VIGNEAUD bekannt. Der Nachteil dieser bekannten Pedikelschrauben ist, dass trotz dem Freiheitsgrad zwischen Pedikelschraube und Längsträger, Letzterer aufgrund der anatomischen Gegebenheiten oft dreidimensional angebogen werden muss.
Bei anderen Pedikelschrauben, wie zum Beispiel gemäss der DE-C 195 09 332 HARMS, wurde dieser Nachteil behoben. Dank der räumlichen Beweglichkeit des Schraubenkopfes gegenüber dem Schraubenteil, muss bei Montagen bis zu drei Pedikelschrauben pro Längsträger der Längsträger nur noch in einer Ebene angebogen werden. Der Nachteil dieser bekannten Pedikelschraube besteht darin, dass Kopf- und Gewindeteil nicht separierbar sind, Kopfteil und Längsträger gegenüber dem Gewindeteil nicht separat blockierbar sind, aufgrund der einseitigen Ausrichtung der Längsschlitze im Druckelement gemäss der DE-C 195 09 332 die erreichbare Festigkeit gering ist und es schwierig ist, den Kopfteil zu remobilisieren, wenn er einmal fixiert ist. Separierbarkeit von Kopf- und Gewindeteil ist ein Bedürfnis, um je nach Bedarf unterschiedliche Kopfteile einsetzen zu können und beim Eindrehen des Schraubenteils in den Knochen die Sicht nicht durch den Kopfteil zu behindern. Ein separates Blockieren von Kopfteil und Längsträger und hohe Festigkeit zwischen Kopfund Gewindeteil ermöglichen das Ausüben von Distraktion und Kompression ohne Winkelverlust zwischen Pedikelschraube und Längsträger und das Aufrechterhalten der anatomischen Krümmung der stabilisierten Wirbelsegmente. Remobilisierung ist vorallem bei der Explantation wie auch bei Neueinstellung der Fixation von Bedeutung.

Eine weitere solche Verbindungsvorrichtung ist in der WO 97/02786 ERRICO offenbart, die es auf einfache Weise gestattet, eine Pedikelschraube oder allgemeiner ein Knochenverankerungselement mit einem Längsträger zu verbinden und die eine grosse Freiheit bezüglich des Winkels zwischen den beiden Elementen gestattet. Bei dieser bekannten Verbindungsvorrichtung besteht der Nachteil darin, dass die Spannzange Bestandteil des Verbindungselementes ist und daher aus demselben Material besteht, was die Materialwahl und -paarung mit der Knochenschraube, zur Erhöhung der Festigkeit, beeinträchtigt.

Hier will die Erfindung Abhilfe schaffen. Das Ziel der Erfindung liegt darin, eine Vorrichtung zur Verbindung eines Längsträgers mit einer Pedikelschraube mit folgenden Eigenschaften zu schaffen:
- einfache Handhabung;
- Eindrehen des Schraubenteils der Vorrichtung auch ohne das Verbindungsteil;
- mögliches nachträgliches Aufklicken des Verbindungsteiles;
- je nach Situation und Vorliebe des Arztes freie Wahl von oder Mischung zwischen seitlich offenen, oben offenen oder geschlossenen Verbindungsteilen. z.B. nach oben offenes Verbindungsteil erleichtert das Einlegen des Längsträgers, währenddem eine seitliche Öffnung seitliche Korrekturen ermöglicht und dank der Möglichkeit des seitlichen Wegkippens des Verbindungsteils die einzelnen Pedikelschrauben jederzeit am Längsträger befestigt oder wieder entfernt werden können, ohne dass das gesamte Fixationssystem demontiert werden muss.

Die Erfindung löst die gestellte Aufgabe mit einer Vorrichtung, welche die Merkmale des Anspruchs 1 aufweist.

Die erfindungsgemässe Vorrichtung besteht aus einem den Längsträger aufnehmenden Verbindungsteil, in welches eine, einen vorzugsweise kugeligen Kopf aufweisende Pedikelschraube nachträglich eingeklickt und fixiert werden kann. Das Verbindungsteil lässt sich in der gewünschten Anzahl und Variante einfach auf die bereits in den Wirbelkörpern implantierten Pedikelschrauben mit Kugelkopf aufklicken, so dass eine primäre Verbindung zwischen Längsträger und Pedikelschraube hergestellt wird. Durch das Einschrauben des Spannmittels in die Fixationsvorrichtung wird gleichzeitig der Längsträger in der Vorrichtung axial und rotativ blockiert und die Vorrichtung winkelstabil fixiert. Dabei drückt die als Spannmittel dienende Stellschraube auf den in die Vorrichtung eingeführten Längsträger, dieser drückt auf einen Hohlzylinder, der einen alternierend geschlitzten, radial nicht fixierten Hohlkegelstumpf umschliesst, welcher seinerseits aufgrund der konischen Ausbildung der Aussenfläche zusammengepresst wird und dadurch den vorzugsweise kugeligen Kopf der Pedikelschraube festklemmt.

Die erfindungsgemässe Vorrichtung bietet somit gegenüber bekannten Vorrichtungen den Vorteil, dass die Pedikelschrauben nicht nur genau senkrecht zum Längsträger fixierbar sind, sondern eine Abwinkelung von bis zu ± 25° gestatten und auf Grund der Distanz zwischen Rotationszentrum des Verbindungsteiles und der Achse des Längsträgers je nach dessen Dicke zwischen 4 und 10 mm zu einem gewissen Grad auch seitliche Abweichungen zwischen Längsträger und Pedikelschraube ausgeglichen werden können. Diese Eigenschaften ermöglichen die Verwendung von in der Produktion standardmässig vorgebogenen Längsträgern, was bei herkömmlichen Systemen zu grossen Schwierigkeiten bei der Montage führt. Ein weiterer Vorteil einer Variante der erfindungsgemässen Vorrichtung besteht darin, dass je nach Ausführung des Hohlzylinders und des Spannmittels, bzw. Ausführung der Vorrichtung Längsträger und der vorzugsweise kugelige Kopf der Knochenschraube separat blockiert werden können.

Die Winkelstabilität kann noch dadurch erhöht werden, dass der Schraubenkopf aus einem relativ harten (z.B. Titan-Aluminium-Niob-Legierung) und der alternierend geschlitzte Hohlkegelstumpf aus einem relativ weichen Material (z.B. Titan in weichem Zustand) besteht.

Eine andere Möglichkeit zur Erhöhung der Winkelstabilität besteht darin, den vorzugsweise kugeligen Kopf der Pedikelschraube und/oder die komplementäre Kavität im Hohlkegelstumpf mit einer dreidimensionalen Strukturierung, z.B. in Form von Rillen zu versehen.

Dadurch wird die auf dem Schraubenkopf angebrachte Strukturierung aus relativ hartem Material in das relativ weiche Material des Hohlkegelstumpf gedrückt. Eine Umkehrung der Härte der Materialien ist mindestens im Kontaktbereich auch möglich. Der Härteunterschied kann auch durch unterschiedliche Kaltverformung oder unterschiedliche Auskristallisation des gleichen Materials erreicht werden, wobei das harte Material vorzugsweise hochtrainierter 1.4441 Stahl ist und das weiche Material warmbehandelter 1.4441 Stahl ist. Daneben kann der Härteunterschied durch Oberflächenbehandlung wie Beschichtung oder Ionenimplantation erreicht werden.

Um die Pedikelschrauben in den Knochen eindrehen zu können, sind sie im Kugelkopf vorzugsweise mit einem Innensechskant versehen. Wenn der aufklickbare Kopf noch durchbohrt ist, kann wahlweise nur die Pedikelschraube oder gleich die gesamte Vorrichtung eingedreht werden. Letzteres hat vor allem den Vorteil, dass jederzeit die Vorrichtung weiter eingedreht oder zurückgedreht werden kann, um einen Höhenausgleich zu erreichen.

Eine Weiterbildung der Erfindung besteht darin, dass die Konuswinkel der Spannzange und des die Spannzange zusammenpressenden Einsatzes verschieden sind und eine nicht selbsthemmende Konusverbindung bilden.

Andere Ausführungsarten der erfindungsgemässen Vorrichtung erlauben ein nachträgliches Einlegen des Längsträgers von der Seite oder von oben. Durch das Einschrauben der Spannschraube in die Fixationsvorrichtung wird gleichzeitig der Längsträger in der Vorrichtung axial und rotativ blockiert und die Vorrichtung winkelstabil fixiert. Dabei drückt die Spannschraube auf den in die Vorrichtung eingeführten Längsträger, dieser drückt auf den Einsatz der Fixationsvorrichtung und dieser Einsatz verspannt über eine vorzugsweise konisch ausgebildete Innenfläche und eine dazu korrespondierende, ebenfalls konische Aussenfläche der Spannzange die federnden Zungen der Spannzange auf dem vorzugsweise kugeligen Kopf der Pedikelschraube.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch den Verbindungsteil einer Variante der erfindungsgemässen Vorrichtung zusammen mit einem Längsträger und einer Pedikelschraube mit Kugelkopf; und
Fig. 2 eine perspektivische Darstellung einer Variante der erfindungsgemässen Vorrichtung.
Fig. 3 einen Längsschnitt durch eine modifizierte erfindungsgemässe Vorrichtung mit Eigenschaften wie in Fig. 1 offenbart, mit dem Unterschied, dass der Längsträger durch einen seitlich offenen Kanal im Verbindungsteil aufgenommen wird; und
Fig. 4 einen Längsschnitt durch eine modifizierte erfindungsgemässe Vorrichtung mit Eigenschaften wie in Fig. 1 offenbart, mit dem Unterschied, dass der Längsträger in einem ovalen Kanal im Verbindungsteil aufgenommen wird;
Fig. 5 eine perspektivische Ansicht einer Variante der erfindungsgemässen Vorrichtung;
Fig. 6 eine perspektivische Ansicht einer weiteren Variante der erfindungsgemässen Vorrichtung;
Fig. 7 eine perspektivische Ansicht einer weiteren Variante der erfindungsgemässen Vorrichtung;
Fig. 8 eine perspektivische Ansicht einer weiteren Variante der erfindungsgemässen Vorrichtung; und
Fig. 9 eine perspektivische Ansicht einer weiteren Variante der erfindungsgemässen Vorrichtung.

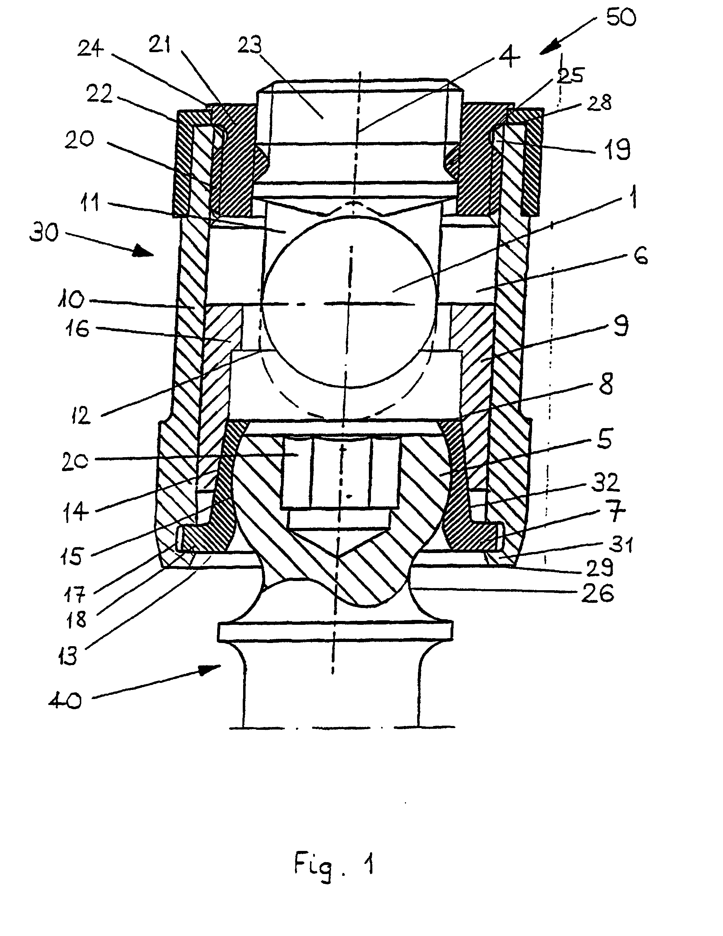

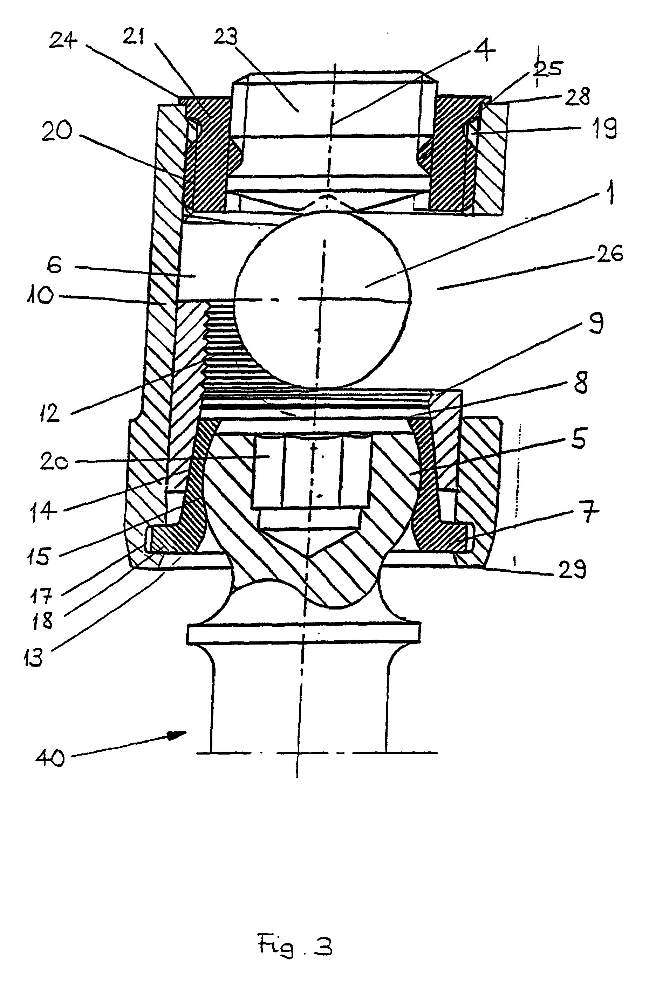

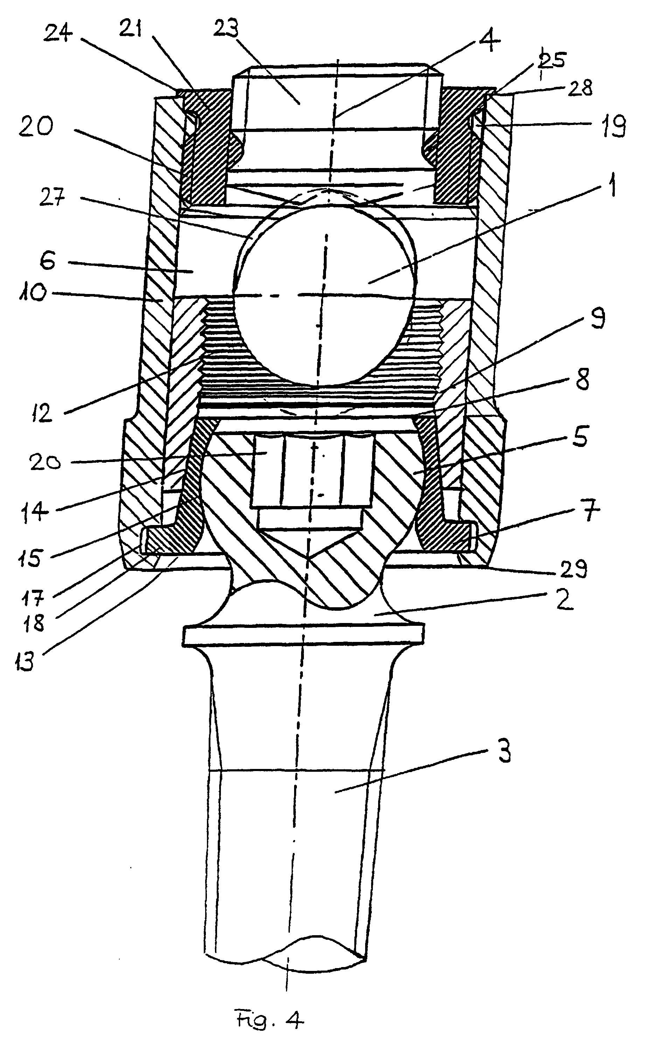

Die in Fig. 1 dargestellte Variante der erfindungsgemässen Vorrichtung besteht im wesentlichen aus einer hohlzylindrischen Hülse 10, die zur Aufnahme des Längsträgers 1 mit einem gegen das obere Ende 28 offenen Kanal 11 versehen ist. Bei Bedarf kann der Kanal 11 auch zur Seite offen sein (Fig. 3) oder als ovale Bohrung (Fig. 4) gestaltet sein. Am unteren Ende 29 der Hülse 10 ist ein vorzugsweise ringförmiger Kanal 17 angebracht, in den der Flansch 18 der in der Bohrung 6 der Hülse 10 eingebrachten Spannzange 7 einhängbar ist. Damit ist die Spannzange 7 gegen Verschiebungen entlang der Zentralachse 4 der Hülse 10 aber nicht radial fixiert. Die Spannzange 7 ist innen mit einer vorzugsweise hohlkugelförmigen Kavität 15 ausgebildet. Schlitze, von denen alternierend ein Anzahl in die obere Basisfläche 8 und eine andere Anzahl in die untere Basisfläche 13 der Spannzange 7 münden, gestatten ein gleichmässiges radiales Ausdehnen und Zusammenpressen der Spannzange 7. Dank der radialen Freiheit der Spannzange 7 im Kanal 17 kann der Kopf 5 der Pedikelschraube 2 jederzeit in die Spannzange 7 ein- und ausgeklinkt werden, solange die Vorrichtung nicht durch Anziehen der Spannschraube 23 blockiert ist. Aussen ist die Spannzange 7 gegen ihr oberes Ende 8 hin sich verjüngend konisch ausgebildet. Ein hohlzylindrischer Einsatz 9, der innen an seinem unteren Ende zum Konus an der Spannzange 7 komplementär konisch ausgebildet ist, kann in der Bohrung 6 in der Hülse 10 gleiten und je nach Krafteinwirkung über die konische Verbindung 14 die Spannzange 7 radial zusammenpressen und damit den sich in der Kavität 15 befindlichen Kopf 5 der Pedikelschraube 2 blockieren. Diese Verbindung ist auch lösbar, was durch das Anbringen eines Absatzes 16 im Einsatz 9 vereinfacht wird. Blockiert werden Längsträger 1 und Pedikelschraube 2 mittels einer Spannschraube 23, welche konzentrisch in eine am oberen Ende 28 der Hülse 10 befindliche Einsatzschraube 21 eingeschraubt wird. Die Spannschraube 23 drückt, wenn sie angezogen wird, auf den Längsträger 1, welcher wiederum auf den Einsatz 9 drückt und damit durch das Ineinanderschieben der Konusse 14 das Schliessen der Spannzange 7 bewirkt.

Die Funktion der Einsatzschraube besteht darin, das durch den Kanal 11 unterbrochene Gewinde 19 in der Hülse 10 in ein geschlossenes Gewinde umzuwandeln, in dem die Spannschrauben 23 ungehindert laufen kann.

Da bei der in Fig. 1 dargestellten Variante der erfindungsgemässen Vorrichtung die Durchgangsöffnung 11 als am oberen Ende 28 der Hülse 10 offener Kanal ausgebildet ist und daher die Hülse 10 an dieser Stelle geschwächt ist, wird ein Überwurfring 22 montiert, der ein Auf spreizen der Hülse 10 beim Anziehen der Spannschraube 23 verhindert.

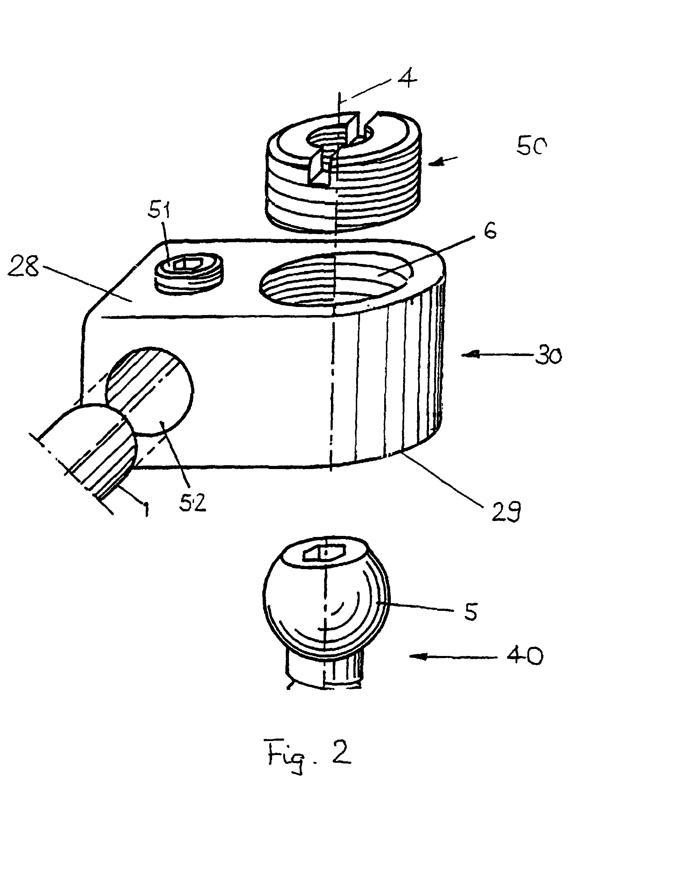

Eine weitere Variante der erfindungsgemässen Vorrichtung ist in Fig. 2 dargestellt.

Der Hauptunterschied zwischen den Ausführungen nach Fig. 1 und Fig. 2 besteht in der seitlichen Versetzung des Kanals 52 zur Aufnahme des Längsträgers 1 gegenüber der Bohrung 6 mit der Zentralachse 4. Dadurch ist es möglich den Längsträger 1 und die Pedikelschraube 2 separate zu fixieren. Vom unteren Ende 29 des Verbindungsteiles 30 her kann ein Knochenverankerungselement 40 eingeführt und analog zu der in Fig. 1 gezeigten Variante mittels einer Spannzange 7, einem Einsatz 9 und eines Spannmittels 50 fixiert werden. Dabei drückt in der in Fig. 2 gezeigten Variante das Spannmittel 50 direkt auf den Einsatz 9. Zur Aufnahme des Längsträgers 1 dient ein quer zur Zentralachse 4 verlaufender, die Bohrung 6 nicht berührender Kanal 52, der wiederum nach bedarf seitlich offen, zum oberen Ende 28 hin offen oder auch als Bohrung ausgeführt sein kann. Die Fixation des Längsträgers 1 geschieht durch eine separate Schraube 51.

Die in Fig. 3 dargestellte Variante der erfindungsgemässen Vorrichtung unterscheidet sich von der in Fig. 1 dargestellten Variante nur dadurch, dass eine seitlich offene Durchgangsöffnung 26 in der Hülse 10 den Längsträger 1 aufnimmt. Der Einsatz 9 ist ebenfalls auf einer Seite tiefer. Damit kann der Längsträger 1 auch wie bei der in Fig. 1 dargestellten Variante nachträglich in die Vorrichtung eingeführt werden.

Die in Fig. 4 dargestellte Variante der erfindungsgemässen Vorrichtung unterscheidet sich von der in Fig. 1 dargestellten Variante nur dadurch, dass eine ovale, geschlossene Durchgangsöffnung 27 in der Hülse 10 den Längsträger 1 aufnimmt.

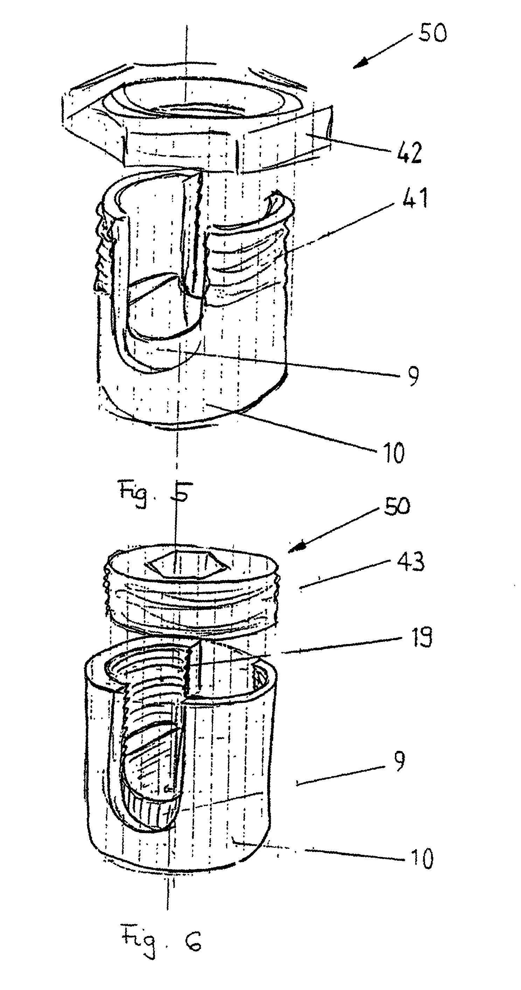

Die in Fig. 5 dargestellte Variante der erfindungsgemässen Vorrichtung unterscheidet sich von der in Fig. 1 dargestellten Variante nur dadurch, dass als Spannmittel 50 eine Mutter 42 verwendet wird. Dazu ist die Hülse 10 mit einem Aussengewinde 41 versehen über welches die Mutter 42 geschraubt wird. Die Mutter 42 drückt, wenn sie angezogen wird, auf den Längsträger 1, der wiederum auf den Einsatz 9 drückt, wodurch die Spannzange 7 radial zusammengepresst und somit der Kopf 5 der Pedikelschraube 2 und der Längsträger gleichsam fixiert werden. Ein Kollabieren des Kanals 11 kann verhindert werden, indem das Gewinde 41 sägezahnförmig ausgebildet ist oder ein zylindrisches Element 47 in die Bohrung 6 eingesetzt wird.

In Fig. 6 wird eine weitere Variante der erfindungsgemässen Vorrichtung dargestellt. Diese unterscheidet sich von der in Fig. 1 dargestellten Variante nur dadurch das als Spannmittel 50 eine Stellschraube 43 dient. Ein Aufweiten des Kanals 11 wird durch die sägezahnförmige Ausbildung des Gewindes 19 verhindert.

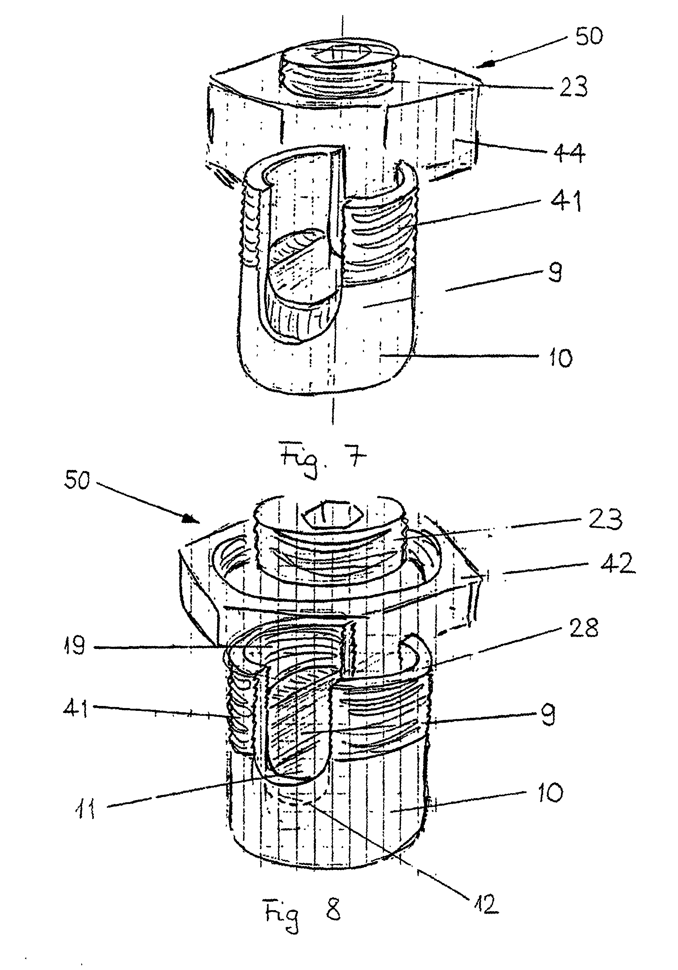

Auch die in Fig. 7 gezeigt Variante unterscheidet sich von der in Fig. 1 dargestellten Variante nur durch die Ausbildung des Spannmittels 50. Die Hülse 10 ist mit einem Aussengewinde 41 versehen über das die Gewindehülse 44 geschraubt wird. Die Fixierung von Längsträger und Pedikelschraube erfolgt über die Spannschraube 23, welche in die Gewindehülse 44 eingeschraubt wird. Die Gewindehülse 44 hat dabei wieder die gleiche Funktion wie der Gewindeeinsatz 21 in Fig. 1. Dank der zwischen dem geschlitzten Gewinde 41 und der Spannschraube 23 eingeschobenen Gewindehülse läuft die Spannschraube 23 in einem intakten, nicht unterbrochenen Gewinde.

Ebenfalls eine weitere Variante der erfindungsgemässen Vorrichtung zeigt Fig. 8. Diese Variante zeichnet sich dadurch aus, dass Längsträger 1 und Pedikelschraube 2 separat fixiert werden können. Dazu ist die Hülse 10 an ihrem oberen Ende 28 mit einem Innengewinde 19 und einem Aussengewinde 41 versehen. Der Kanal 12 des Einsatzes 9 ist so gestaltet, dass der Längsträger 1 bei dessen Fixierung mittels der Mutter 42 immer auf dem unteren Rand der Durchgangsöffnung 11 aufliegt. Beim Anziehen der Spannschraube 23, welche direkt auf den über den Längsträger 1 reichenden Einsatz 9 drückt, wird dann die Spannzange 7 zusammengepresst, womit die Pedikelschraube 2 blockiert wird.

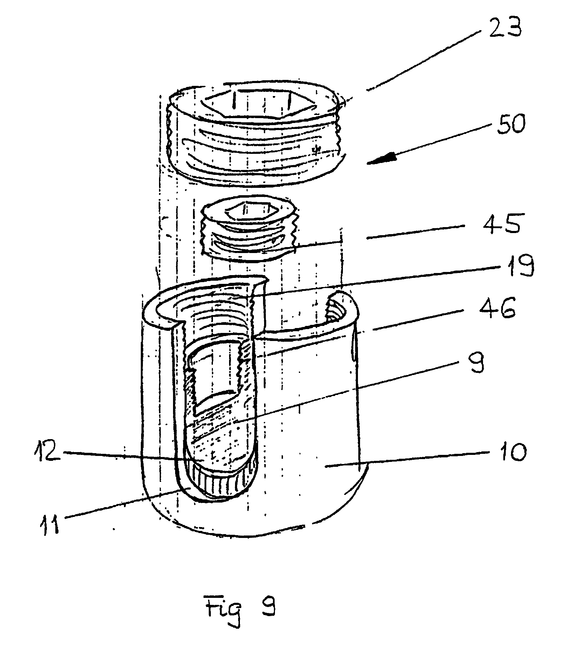

Auch die in Fig. 9 dargestellte Variante der erfindungsgemässen Vorrichtung gestattet ein separates Blockieren von Längsträger 1 und Pedikelschraube 2. Die Durchgangsöffnung 11 und der Kanal 12 sind so gestaltet, dass der Längsträger 1 bei dessen Fixierung immer auf dem Kanal 12 des Einsatzes 9 aufliegt. Blockiert wird der Längsträger 1 mittels einer Stiftschraube 45, die in ein Innengewinde 46 im Einsatz 9 eingeschraubt wird. Zur Fixierung der Pedikelschraube 2 dient eine Spannschraube 23, die auf den Einsatz 9 drückt, wodurch sich dieser über die Spannzange 7 schiebt und diese radial zusammenpresst. Auch hier kann das Aufweiten des Kanals 11 entweder durch sägezahnförmige Ausbildung der Gewinde 19 und 46 oder durch eine wie in Fig. 1 gezeigte Hülse 22 verhindert werden.

## Patentansprüche

1. Vorrichtung zur Verbindung eines Längsträgers (1) mit einem Knochenverankerungselement (40) innerhalb eines Wirbelsäulenfixationssystems, mit
A) einem Verbindungsteil (10;30) mit einem oberen Ende (28), einem unteren Ende (29) und einer gegen das untere Ende (29) hin offenen Bohrung (6) mit einer Zentralachse (4) und einer quer zur Zentralachse (4) verlaufenden Durchgangsöffnung (11) zur Aufnahme eines Längsträgers (1),
B) einem am oberen Ende (28) einsetzbaren Spannmittel (50) zur Fixation des Knochenverankerungselementes (40) und
C) einer im Verbindungsteil (10;30) koaxial zur Zentralachse (4) angeordneten, radial zusammendrückbaren Spannzange (7) mit einem oberen Ende (8) und einem unteren Ende (13), welche innen mit einer in Richtung der Zentralachse (4) nach unten offenen Kavität (15) zur federnden Aufnahme des Kopfes (5) eines Knochenverankerungselementes (40) ausgebildet ist,
**dadurch gekennzeichnet, dass**
D) die Spannzange (7) in Richtung gegen das untere Ende (29) hin axial in der Bohrung (6) nahe dem unteren Ende (29) des Verbindungsteiles (10;30) abgestützt ist, ohne dadurch die Spannzange (7) in ihrer radialen Beweglichkeit zu behindern; und
E) dass ein in der Bohrung (6) des Verbindungsteiles (10; 30) koaxial gleitbarer Einsatz (9) mit einer zur Aussenfläche (32) der Spannzange (7) komplementären Bohrung (14) vorgesehen ist.

2. Vorrichtung nach Anspruch 1, **dadurch gekennzeichnet, dass** durch einen rechtwinklig zur Zentralachse (4) verlaufenden Absatz (31) in der Bohrung (6), vorzugsweise in Form einer Ringnut (17), die Spannzange (7) axial gesichert ist.

3. Vorrichtung nach einem der Ansprüche 1 oder 2, **dadurch gekennzeichnet, dass** das Verbindungsteil (10) eine hohlzylindrische Hülse (10) ist.

4. Vorrichtung nach einem der Ansprüche 1 bis 3, **dadurch gekennzeichnet, dass** die Aussenfläche (32) der Spannzange (7) konisch ausgebildet ist.

5. Vorrichtung nach einem der Ansprüche 1 bis 4, **dadurch gekennzeichnet, dass** die Bohrung (14), nach unten erweitert, konisch ausgebildet ist.

6. Vorrichtung nach Anspruch 4 oder 5, **dadurch gekennzeichnet, dass** der Kegelwinkel der konischen ausgebildeten Aussenfläche (32) der Spannzange (7) und der Kegelwinkel der konisch ausgebildeten Bohrung (14) im Einsatz (9) grösser als 10° ist und vorzugsweise zwischen 12° und 16° liegt.

7. Vorrichtung nach einem der Ansprüche 1 bis 6, **dadurch gekennzeichnet, dass** der Kegelwinkel der konischen ausgebildeten Aussenfläche (32) der Spannzange (7) und der Kegelwinkel der konisch ausgebildeten Bohrung (14) im Einsatz (9) eine Differenz aufweisen, welche vorzugsweise kleiner als 1° ist.

8. Vorrichtung nach Anspruch 7, **dadurch gekennzeichnet, dass** die Differenz grösser als 1° ist und vorzugsweise zwischen 4° und 6° liegt.

9. Vorrichtung nach einem der Ansprüche 1 bis 8, **dadurch gekennzeichnet, dass** eine seitlich offene Durchgangsöffnung (26) im Verbindungsteil (10;30) zur Aufnahme des Längsträgers (1) dient.

10. Vorrichtung nach einem der Ansprüche 1 bis 8, **dadurch gekennzeichnet, dass** eine ovale Durchgangsöffnung (27) im Verbindungsteil (10;30) zur Aufnahme des Längsträgers (2) dient.

11. Vorrichtung nach einem der Ansprüche 1 bis 10, **dadurch gekennzeichnet, dass** das Innengewinde (19) im Verbindungsteil (10:30) und das Aussengewinde (20) der Einsatzschraube (21) als Sägezahngewinde ausgebildet sind, wobei die flachen Profilseiten des Innengewindes (19) gegen die Spannzange (7) gerichtet sind und zur Zentralachse (4) einen Winkel zwischen 87° und 93° aufweisen.

12. Vorrichtung nach einem der Ansprüche 1 bis 11, **dadurch gekennzeichnet, dass** als Knochenverankerungselement (40) eine Pedikelschraube (2) dient, wobei sich der Durchmesser des Gewindeschaftes (3) gegen sein endständiges, freies Teil hin konisch verjüngen kann, vorzugsweise mit einem Konuswinkel von 3° - 4°.

13. Vorrichtung nach einem der Ansprüche 1 bis 12, **dadurch gekennzeichnet, dass** das Knochenverankerungselement (40) unterhalb des kugeligen Kopfes (5) mit einer Eindrehung (53) versehen ist.

14. Vorrichtung nach einem der Ansprüche 1 bis 13, **dadurch gekennzeichnet, dass** der Hohlraum (14) im Einsatz (9) im Bereich der Kontaktzone zwischen Einsatz (9) und Spannzange (7) konvex geformt ist.

15. Vorrichtung nach einem der Ansprüche 1 bis 14, **dadurch gekennzeichnet, dass** die Spannzange (7) im Bereich der Kontaktzone zwischen Einsatz (9) und Spannzange (7) konvex, vorzugweise kugelig geformt ist.

16. Vorrichtung nach einem der Ansprüche 1 bis 15, **dadurch gekennzeichnet, dass** der Einsatz (9) im oberen Bereich eine Verankerungsmöglichkeit, vorzugsweise einen Absatz (16) aufweist.

17. Vorrichtung nach einem der Ansprüche 1 bis 16, **dadurch gekennzeichnet, dass** der Einsatz (9) kreiszylindrisch ist.

18. Vorrichtung nach einem der Ansprüche 1 bis 16, **dadurch gekennzeichnet, dass** der Einsatz (9) quer zur Zentralachse (4) einen polygonartigen Querschnitt aufweist.

19. Vorrichtung nach einem der Ansprüche 1 bis 18, **dadurch gekennzeichnet, dass** der Einsatz (9) innen auch gegen oben offen ist.

20. Vorrichtung nach einem der Ansprüche 1 bis 19, **dadurch gekennzeichnet, dass** der Einsatz (9) im oberen Bereich einen Kanal (12) aufweist.

21. Vorrichtung nach einem der Ansprüche 1 bis 20, **dadurch gekennzeichnet, dass** die Bohrung (6) und die Durchgangsöffnung (11) miteinander verbunden sind.

22. Vorrichtung nach einem der Ansprüche 1 bis 21, **dadurch gekennzeichnet, dass** das Spannmittel (50) ein gleichzeitiges Fixieren von Längsträger (1) und Kopf (5) des Knochenverankerungselementes (40) erlaubt.

23. Vorrichtung nach einem der Ansprüche 1 bis 22, **dadurch gekennzeichnet, dass**
A) das Spannmittel (50) direkt auf den Längsträger (1) drückt; und
B) die Bewegung des Längsträgers (1) beim Spannvorgang nicht durch die Durchgangsöffnung (11) in der Hülse (10) behindert wird.

24. Vorrichtung nach Anspruch 23, **dadurch gekennzeichnet, dass** das obere Ende (28) der Hülse (10) ein Aussengewinde (41) aufweist, auf das eine Mutter (42) als Spannmittel (50) aufgedreht wird.

25. Vorrichtung nach Anspruch 23, **dadurch gekennzeichnet, dass** das obere Ende (28) der Hülse (10) ein Innengewinde (19) aufweist, in das eine Stellschraube (43) als Spannmittel (50) eingedreht wird.

26. Vorrichtung nach einem der Ansprüche 1 bis 25, **dadurch gekennzeichnet, dass** das Spannmittel (50) mehrteilig ist.

27. Vorrichtung nach Anspruch 23, **dadurch gekennzeichnet, dass** eine Einsatzschraube (21) mit integrierter Spannschraube (23) als Spannmittel (50) dient.

28. Vorrichtung nach Anspruch 23, **dadurch gekennzeichnet, dass** eine Gewindehülse (44) mit integrierter Spannschraube (23) als Spannmittel (50) dient.

29. Vorrichtung nach einem der Ansprüche 1 bis 22, **dadurch gekennzeichnet, dass** das obere Ende (28) der Hülse (10) ein Aussengewinde (41) aufweist, auf das eine Mutter (42) zur Fixierung des Längsträgers (1) aufgedreht wird.

30. Vorrichtung nach einem der Ansprüche 1 bis 29, **dadurch gekennzeichnet, dass**
A) eine Mutter (42) direkt auf den Längsträger (1) drückt; und
B) die Bewegung des Längsträgers (1) beim Spannvorgang nicht durch den Kanal (12) behindert wird.

31. Vorrichtung nach Anspruch 30, **dadurch gekennzeichnet, dass**
A) das obere Ende (28) der Hülse (10) ein Innengewinde (19) aufweist und
B) der Einsatz (9) über den Längsträger (1) reicht, so dass beim Anziehen der Spannschraube (23) der Einsatz (9) nach unten bewegt wird, ohne auf den Längsträger (1) zu drücken.

32. Vorrichtung nach einem der Ansprüche 1 bis 22, **dadurch gekennzeichnet, dass** der Einsatz (9) oben ein Innengewinde (46) aufweist.

33. Vorrichtung nach Anspruch 32, **dadurch gekennzeichnet, dass**
A) eine Stiftschraube (45) auf den Längsträger (1) drückt; und
B) die Bewegung des Längsträgers (1) beim Spannvorgang nicht durch die Durchgangsöffnung (11) behindert wird.

34. Vorrichtung nach einem der Ansprüche 1 bis 33, **dadurch gekennzeichnet, dass** die Spannzange (7) innen mit einer hohlkugelförmigen Kavität (15) zur Aufnahme des vorzugsweise kugeligen Kopfes (5) des Knochenverankerungselementes (40) versehen ist.

35. Vorrichtung nach einem der Ansprüche 1 bis 34, **dadurch gekennzeichnet, dass** die Spannzange (7) mindestens vier Schlitze aufweist, von denen eine Anzahl in die Stirnfläche am unteren Ende (13) münden.

36. Vorrichtung nach einem der Ansprüche 1 bis 34, **dadurch gekennzeichnet, dass** die Spannzange (7) mindestens vier Schlitze aufweist, von denen eine Anzahl in die Stirnfläche am oberen Ende (8) münden.

37. Vorrichtung nach einem der Ansprüche 1 bis 34, **dadurch gekennzeichnet, dass** die Spannzange (7) mindestens vier Schlitze aufweist, die alternierend in die Stirnfläche am unteren Ende (13) und in die Stirnfläche am oberen Ende (8) münden.

38. Vorrichtung nach einem der Ansprüche 1 bis 23, 25 bis 27 und 32 bis 37, **dadurch gekennzeichnet, dass** ein Überwurfring (22) am oberen Ende (28) der Hülse (10) das Aufspreizen der Hülse (10) vermeidet.

39. Vorrichtung nach Anspruch 38, **dadurch gekennzeichnet, dass** der Überwurfring (22) durch eine Einsatzschraube (21) an der Hülse (10) befestigt werden kann.

40. Vorrichtung nach einem der Ansprüche 1 bis 39, **dadurch gekennzeichnet, dass**
A) der Kanal (11) in der Hülse (10) gegen das obere Ende (28) hin offen ist;
B) die Gewinde (19;41) am oberen Ende (28) der Hülse (10) und die komplementären Gewinde des entsprechenden Spannmittels (50) sägezahnartig ausgebildet sind, wobei die flachen Profilseiten derart gerichtet sind, dass beim Anziehen des Spannmittels (50) kein Spreizen der Hülse (10) stattfindet; und
C) die flache Profilseite einen Winkel zwischen 87° und 93° zur Zentralachse (4) aufweist.

41. Vorrichtung nach einem der Ansprüche 1 bis 21 und 33 bis 37, **dadurch gekennzeichnet, dass** im Verbindungsteil (30) ein Kanal (52) quer zur Zentralachse (4) und neben der Bohrung (6) versetzt zur Aufnahme und eine Schraube (51) zur Fixierung des Längsträgers (1) dient.

42. Vorrichtung gemäss einem der Ansprüche 1 bis 41, **dadurch gekennzeichnet, dass** die Kavität (15) in der Spannzange (7) und/oder der Kopf (5) des Knochenverankerungselementes (40) mit einer Strukturierung, vorzugsweise in Form von Querrillen, bzw. Querrippen versehen ist.

43. Vorrichtung nach einem der Ansprüche 1 bis 42, **dadurch gekennzeichnet, dass** die Spannzange (7) und der Kopf (5) des Knochenverankerungselementes (40) mindestens im Kontaktbereich aus Materialien mit unterschiedlicher Härte bestehen.

44. Vorrichtung nach Anspruch 43, **dadurch gekennzeichnet, dass** das härtere Material vorzugsweise Titan-Aluminium-Niob ist und das weiche Material vorzugsweise weiches Reintitan ist.

45. Vorrichtung nach einem der Ansprüche 1 bis 42, **dadurch gekennzeichnet, dass** die Spannzange (7) und der Kopf (5) des Knochenverankerungselementes (40) mindestens im Kontaktbereich eine verschiedene Härte aufweisen, wobei dieser Härteunterschied durch unterschiedliche Kaltverformung oder unterschiedliche Auskristallisation des gleichen Materials erreicht wird und dabei das harte Material vorzugsweise hochtrainierter 1.4441 Stahl ist und das weiche Material warmbehandelter 1.4441 Stahl ist.

46. Vorrichtung nach einem der Ansprüche 1 bis 42, **dadurch gekennzeichnet, dass** die Spannzange (7) und der Kopf (5) des Knochenverankerungselementes (40) mindestens im Kontaktbereich eine verschiedene Härte aufweisen, wobei dieser Härteunterschied durch Oberflächenbehandlung wie Beschichtung oder Ionenimplantation erreicht wird.

47. Vorrichtung nach einem der Ansprüche 1 bis 46, **dadurch gekennzeichnet, dass** durch einen rechtwinklig zur Zentralachse (4) verlaufenden Absatz (31) in der Bohrung (6) die Spannzange (7) axial gegen das untere Ende (29) hin gesichert ist.

48. Vorrichtung nach einem der Ansprüche 1 bis 47, **dadurch gekennzeichnet, dass** die Spannzange (7) axialfest in der Bohrung (6) abgestützt ist.

## Claims

1. Device for the connection of a longitudinal rod (1) with a bone anchoring element (40) within a vertebral fixation system, with
A) a connection part (10;30) with an upper end (28), a lower end (29) and a bore hole (6) open towards the lower end (29) having a central axis (4) and a through hole (11) running transversely to the central axis (4) for the acceptance of a longitudinal rod (1),
B) a tension means (50) for the fixation of the bone anchoring element (40) insertable from the upper end (28) and
C) a radially compressible spring chuck (7) arranged within the connection part (10;30) coaxial to the central axis (4) with an upper end (8) and a lower end (13) which is provided in its inside with a cavity (15) downwardly and in the direction of the central axis (4) open for the springlike acceptance of the head (5) of a bone anchoring element (40),
**characterized in that**
D) the spring chuck (7) is axially supported in the direction towards the lower end (29) in the bore hole (6) close to the lower end (29) of the connection part (10;30) without impeding the spring chuck (7) in its radial movability therewith; and
E) an insert (9) is provided coaxially slidable in the bore hole (6) of the connection part (10;30) having a bore (14) complementary to the outer surface (32) of the spring chuck (7).

2. Device according to claim 1, **characterized in that** the spring chuck (7) is axially secured by means of a shoulder (31) preferably in the shape of a ring groove running perpendicular to the central axis (4) in the bore hole (6).

3. Device according to one of the claims 1 or 2, **characterized in that** the connection part (10) is a hollow cylindrical sleeve (10).

4. Device according to one of the claims 1 to 3, **characterized in that** the outer surface (32) of the spring chuck (7) is conically shaped.

5. Device according to one of the claims 1 to 4, **characterized in that** the bore (14) is shaped conically widening downwardly.

6. Device according to claim 4 or 5, **characterized in that** the angle of the cone of the conically shaped outer surface (32) of the spring chuck (7) and the angle of the cone of the conically shaped bore (14) in the insert (9) is greater than 10° and amounts preferably between 12° and 16°.

7. Device according to one of the claims 1 to 6, **characterized in that** the angle of the cone of the conically shaped outer surface (32) of the spring chuck (7) and the angle of the cone of the conically shaped bore (14) in the insert (9) provide a difference that is preferably smaller than 1°.

8. Device according to claim 7, **characterized in that** the difference is greater than 1° and amounts preferably between 4° and 6°.

9. Device according to one of the claims 1 to 8, **characterized in that** a sidewardly open through hole (26) in the connection part (10;30) serves to the acceptance of the longitudinal rod (1).

10. Device according to one of the claims 1 to 8, **characterized in that** an oval through hole (27) in the connection part (10;30) serves to the acceptance of the longitudinal rod (1).

11. Device according to one of the claims 1 to 10, **characterized in that** the interior thread (19) in the connection part (10;30) and the external thread (20) of the insert screw (21) are formed as buttress threads whereby the flat sides of the profile of the interior thread (19) are directed towards the spring chuck (7) and provide an angle to the central axis (4) amounting between 87° and 93°.

12. Device according to one of the claims 1 to 11, **characterized in that** a pedicle screw (2) serves as bone anchoring element (40) whereby the diameter of the threaded shaft (3) may conically taper towards its terminal free section, preferably with an angle of the cone with an amount of 3° - 4°.

13. Device according to one of the claims 1 to 12, **characterized in that** the bone anchoring element (40) is provided with a turned groove (53) below the spherical head (5).

14. Device according to one of the claims 1 to 13, **characterized in that** the hollow space (14) in the insert (9) is convexly shaped in the area of the contact zone between insert (9) and spring chuck (7).

15. Device according to one of the claims 1 to 14, **characterized in that** the spring chuck (7) is convexly preferably spherically shaped in the area of the contact zone between insert (9) and spring chuck (7).

16. Device according to one of the claims 1 to 15, **characterized in that** the insert (9) provides an anchoring means preferably a shoulder (16) in the upper area.

17. Device according to one of the claims 1 to 16, **characterized in that** the insert is circular cylindrical.

18. Device according to one of the claims 1 to 16, **characterized in that** the insert (9) provides a polygon like cross section transversely to the central axis (4).

19. Device according to one of the claims 1 to 18, **characterized in that** the insert (9) is also open on the inside towards the top.

20. Device according to one of the claims 1 to 19, **characterized in that** the insert (9) provides a channel (12) in the upper section.

21. Device according to one of the claims 1 to 20, **characterized in that** the bore hole (6) and the through hole (11) are connected to each other.

22. Device according to one of the claims 1 to 21, **characterized in that** the tension means (50) enables a simultaneous fixation of the longitudinal rod (1) and the head (5) of the bone anchoring element (40).

23. Device according to one of the claims 1 to 22, **characterized in that**
A) the tension means (50) presses directly on the longitudinal rod (1); and
B) the motion of the longitudinal rod (1) is not impeded during the tensioning action through the through hole (11) in the sleeve (10).

24. Device according to claim 23, **characterized in that** the upper end (28) of the sleeve (10) provides an external thread (41) whereon a nut is screwed as tension means (50).

25. Device according to claim 23, **characterized in that** the upper end (28) of the sleeve (10) provides an internal thread (19) wherein a locking screw (43) is screwed as tension means (50).

26. Device according to one of the claims 1 to 25, **characterized in that** the tension means (50) is multipiece.

27. Device according to claim 23, **characterized in that** an insert screw (21) with integrated tension screw (23) serves as tension means (50).

28. Device according to claim 23, **characterized in that** a threaded sleeve (44) with integrated tension screw (23) serves as tension means (50).

29. Device according to one of the claims 1 to 22, **characterized in that** the upper end (28) of the sleeve (10) provides an external thread (41) whereon a nut (42) is screwed to the fixation of the longitudinal rod (1).

30. Device according to one of the claims 1 to 29, **characterized in that**
A) a nut presses directly on the longitudinal rod (1); and
B) the motion of the longitudinal rod (1) is not impeded during the tensioning action through the channel (12).

31. Device according to claim 30, **characterized in that**
A) the upper end (28) of the sleeve (10) provides an internal thread (19) and
B) the insert (9) extends over the longitudinal rod (1) so that in case of tightening the tension screw (23) the insert (9) is moved downwards without pressing on the longitudinal rod (1).

32. Device according to one of the claims 1 to 22, **characterized in that** the insert (9) provides an internal thread (46) topside.

33. Device according to claim 32, **characterized in that**
A) a stud bolt (45) presses on the longitudinal rod (1); and
B) the motion of the longitudinal rod (1) is not impeded during the tensioning action through the through hole (11).

34. Device according to one of the claims 1 to 33, **characterized in that** the spring chuck (7) is provided on its inside with a cavity (15) having the form of a hollow sphere to the acceptance of the preferably spherical head (5) of the bone anchoring element (40).

35. Device according to one of the claims 1 to 34, **characterized in that** the spring chuck (7) provides at least four slots whereof a number run into the front side at the lower end (13).

36. Device according to one of the claims 1 to 34, **characterized in that** the spring chuck (7) provides at least four slots whereof a number run into the front side at the upper end (8).

37. Device according to one of the claims to 34, **characterized in that** the spring chuck (7) provides at least four slots which run alternately into the front side at the lower end (13) and into the front side at the upper end (8).

38. Device according to one of the claims 1 to 23, 25 to 27 and 32 to 37, **characterized in that** a swivel ring (22) at the upper end (28) of the sleeve (10) impedes spreading of the sleeve (10).

39. Device according to claim 38, **characterized in that** the swivel ring (22) may be attached at the sleeve (10) by means of an insert screw (21).

40. Device according to one of the claims 1 to 39, **characterized in that**
A) the channel (11) at the sleeve (10) is open towards the upper end (28);
B) the threads (19;41) at the upper end (28) of the sleeve (10) and the complementary threads of the respective tension means (50) are provided as buttress threads whereby the flat sides of the profile are directed such that in case of tightening the tension means (50) no spreading of the sleeve (10) appears; and
C) the flat side of the profile provides an angle having an amount between 87° and 93° to the central axis (4).

41. Device according to one of the claims 1 to 21 and 33 to 37, **characterized in that** a channel (52) in the connection part (30) running transverse to the central axis (4) and dislocated beside the bore hole (6) serves to the acceptance of the longitudinal rod (1) and a screw (51) serves to the fixation of the longitudinal rod (1).

42. Device according to one of the claims 1 to 41, **characterized in that** the cavity (15) within the spring chuck (7) and/or the head (5) of the bone anchoring element (40) is provided with a structure preferably in the form of transverse grooves respectively transverse ribs.

43. Device according to one of the claims 1 to 42, **characterized in that** the spring chuck (7) and the head (5) of the bone anchoring element (40) consist at least in the contact area of materials with different hardness.

44. Device according to claim 43, **characterized in that** the harder material is preferably Titanium-Aluminium-Niobium and the soft material is preferably pure Titanium.

45. Device according to one of the claims 1 to 42, **characterized in that** the spring chuck (7) and the head (5) of the bone anchoring element (40) provide at least in the contact area a different hardness whereby this difference of hardness is obtained via different cold forming or different crystallization of the same material and whereby the harder material is preferably highly trained steel 1.4441 and the soft material is hot-treated steel 1.4441.

46. Device according to one of the claims 1 to 42, **characterized in that** the spring chuck (7) and the head (5) of the bone anchoring element (40) provide at least in the contact area a different hardness, whereby this difference of hardness is obtained via surface treatment as coating or ion implantation.

47. Device according to one of the claims 1 to 46, **characterized in that** the spring chuck (7) is axially secured towards the lower end (28) by means of a shoulder (31) within the bore hole (6) running orthogonal to the central axis (4).

48. Device according to one of the claims 1 to 47, **characterized in that** the spring chuck (7) is supported axially stable within the bore hole (6).

## Revendications

1. Dispositif pour relier un support longitudinal (1) à un élément (40) d'ancrage dans l'os dans un système de fixation de la colonne vertébrale, qui comporte:
A) une pièce de liaison (10; 30) dotée d'une extrémité supérieure (28), d'une extrémité inférieure (29) et d'un alésage (6) ouvert en direction de l'extrémité inférieure (29) et présentant un axe central (4) et une ouverture de passage (11) qui s'étend transversalement à l'axe central (4) pour recevoir un support longitudinal (1);
B) un moyen de serrage (50) qui peut être inséré sur l'extrémité supérieure (28) pour la fixation de l'élément (40) d'ancrage dans l'os, et
C) des mâchoires de serrage (7) disposées dans la pièce de liaison (10; 30) coaxialement à l'axe central (4) et pouvant être comprimées radialement, avec une extrémité supérieure (8) et une extrémité inférieure (13) qui est configuré du côté intérieur avec une cavité (15) ouverte vers le bas en direction de l'axe central (4) pour une réception élastique de la tête (5) d'un élément (40) d'ancrage dans l'os,
**caractérisé en ce que**
D) les mâchoires de serrage (7) s'appuient axialement en direction de l'extrémité inférieure (29) dans l'alésage (6) à proximité de l'extrémité inférieure (29) de la pièce de liaison (10; 30) sans par là gêner la mobilité radiale des mâchoires de serrage (7); et
E) **en ce que** dans l'alésage (6) de la pièce de liaison (10; 30) est prévue une garniture (9) apte à coulisser coaxialement et dotée d'un alésage (14) complémentaire de la surface extérieure (32) des mâchoires de serrage (7).

2. Dispositif selon la revendication 1, **caractérisé en ce que** les mâchoires de serrage (7) sont immobilisées axialement par un épaulement (31) ménagé dans l'alésage (6), qui présente de préférence la forme d'une rainure annulaire (17) et qui s'étend perpendiculairement à l'axe central (4).

3. Dispositif selon l'une des revendications 1 ou 2, **caractérisé en ce que** la pièce de liaison (10) est une douille cylindrique creuse (10).

4. Dispositif selon l'une des revendications 1 à 3, **caractérisé en ce que** la surface extérieure (32) des mâchoires de serrage (7) présente une configuration conique.

5. Dispositif selon l'une des revendications 1 à 4, **caractérisé en ce que** l'alésage (14) évasé vers le bas présente une configuration conique.

6. Dispositif selon la revendication 4 ou 5, **caractérisé en ce que** l'angle du cône de la surface extérieure (32) de configuration conique des mâchoires de serrage (7) et l'angle du cône de l'alésage (14) de configuration conique ménagé dans la garniture (9) est plus grand que 10° et est de préférence compris entre 12° et 16°.

7. Dispositif selon l'une des revendications 1 à 6, **caractérisé en ce que** l'angle du cône de la surface extérieure (32) de configuration conique des mâchoires de serrage (7) et l'angle du cône de l'alésage (14) de configuration conique ménagé dans la garniture (9) présentent une différence qui est de préférence inférieure à 1°.

8. Dispositif selon la revendication 7, **caractérisé en ce que** la différence est supérieure à 1° et est de préférence comprise entre 4° et 6°.

9. Dispositif selon l'une des revendications 1 à 8, **caractérisé en ce qu'**une ouverture de passage (26) ouverte latéralement ménagée dans la pièce de liaison (10; 30) sert à recevoir le support longitudinal (1).

10. Dispositif selon l'une des revendications 1 à 8, **caractérisé en ce qu'**une ouverture de passage ovale (27) ménagée dans la pièce de liaison (10; 30) sert à recevoir le support longitudinal (2).

11. Dispositif selon l'une des revendications 1 à 10, **caractérisé en ce que** le filet intérieur (19) ménagé dans la pièce de liaison (10; 30) et le filet extérieur (20) de la garniture filetée (21) sont configurées comme filets en dents de scie, les côtés plats profilés du filet intérieur (19) étant orientés vers les mâchoires de serrage (7) et présentant par rapport à l'axe central (4) un angle compris entre 87° et 93°.

12. Dispositif selon l'une des revendications 1 à 11, **caractérisé en ce qu'**une vis à pédoncule (2) sert d'élément (40) d'ancrage dans l'os, le diamètre de la tige filetée (3) pouvant se rétrécir coniquement en direction de son extrémité terminale libre, de préférence avec un angle de cône de 3° à 4°.

13. Dispositif selon l'une des revendications 1 à 12, **caractérisé en ce que** l'élément (40) d'ancrage dans l'os est doté d'un dispositif de serrage (53) en dessous de la tête sphérique (5).

14. Dispositif selon l'une des revendications 1 à 13, **caractérisé en ce que** l'espace creux (14) prévu dans la garniture (9) en direction de la zone de contact entre la garniture (9) et les mâchoires de serrage (7) présente une configuration convexe.

15. Dispositif selon l'une des revendications 1 à 14, **caractérisé en ce que** les mâchoires de serrage (7) présentent une forme convexe, de préférence conique dans la région de la zone de contact entre la garniture (9) et les mâchoires de serrage (7).

16. Dispositif selon l'une des revendications 1 à 15, **caractérisé en ce que** la garniture (9) présente dans sa région supérieure une possibilité d'ancrage, de préférence un épaulement (16).

17. Dispositif selon l'une des revendications 1 à 16, **caractérisé en ce que** la garniture (9) est cylindrique circulaire.

18. Dispositif selon l'une des revendications 1 à 16, **caractérisé en ce que** la garniture (9) présente une section polygonale transversalement à l'axe central (4).

19. Dispositif selon l'une des revendications 1 à 18, **caractérisé en ce que** la garniture (9) est ouverte du côté intérieur ainsi que vers le haut.

20. Dispositif selon l'une des revendications 1 à 19, **caractérisé en ce que** la garniture (9) présente un canal (12) dans sa région supérieure.

21. Dispositif selon l'une des revendications 1 à 20, **caractérisé en ce que** l'alésage (6) et l'ouverture de passage (11) sont reliés l'un à l'autre.

22. Dispositif selon l'une des revendications 1 à 21, **caractérisé en ce que** le moyen de serrage (50) permet simultanément la fixation du support longitudinal (1) et de la tête (5) de l'élément (40) d'ancrage dans l'os.

23. Dispositif selon l'une des revendications 1 à 22, **caractérisé en ce que**
A) le moyen de serrage (50) repousse directement le support longitudinal (1) et
B) le déplacement du support longitudinal (1) n'est pas gêné par l'ouverture de passage (11) ménagée dans la douille (10) lors de l'opération de serrage.

24. Dispositif selon la revendication 23, **caractérisé en ce que** l'extrémité supérieure (28) de la douille (10) présente un filet extérieur (40) sur lequel un écrou (42) est serré en tant que moyen de serrage (50).

25. Dispositif selon la revendication 23, **caractérisé en ce que** l'extrémité supérieure (28) de la douille (10) présente un filet intérieur (19) dans lequel une vis de serrage (43) est serrée comme moyen de serrage (50).

26. Dispositif selon l'une des revendications 1 à 25, **caractérisé en ce que** le moyen de serrage (50) est réalisé en plusieurs pièces.

27. Dispositif selon la revendication 23, **caractérisé en ce qu'**une garniture filetée (21) dotée d'une vis de serrage (23) intégrée sert de moyen de serrage (50).

28. Dispositif selon la revendication 23, **caractérisé en ce qu'**une douille filetée (44) avec une vis de serrage (23) intégrée sert de moyen de serrage (50).

29. Dispositif selon l'une des revendications 1 à 22, **caractérisé en ce que** l'extrémité supérieure (28) de la douille (10) présente un filet extérieur (41) sur lequel un écrou (42) est serré pour fixer la support longitudinal (1).

30. Dispositif selon l'une des revendications 1 à 29, **caractérisé en ce que**
A) un écrou (42) repousse directement le support longitudinal (1) et
B) le déplacement du support longitudinal (1) n'est pas gêné par le canal (12) lors de l'opération de serrage.

31. Dispositif selon la revendication 30, **caractérisé en ce que**
A) l'extrémité supérieure(28) de la douille (10) présente un filet intérieur (19) et
B) la garniture (9) déborde au-delà du support longitudinal (1) de sorte que lors du serrage de la vis de serrage (23), la garniture (9) est déplacée vers le bas sans repousser le support longitudinal (1).

32. Dispositif selon l'une des revendications 1 à 22, **caractérisé en ce que** la garniture (9) présente dans le haut un filet intérieur (46).

33. Dispositif selon la revendication 32, **caractérisé en ce que**
A) une vis à tige (45) repousse le support longitudinal (1) et
B) le déplacement du support longitudinal (1) lors de l'opération de serrage n'est pas gêné par l'ouverture de passage (11).

34. Dispositif selon l'une des revendications 1 à 23, **caractérisé en ce que** les mâchoires de serrage (7) sont dotées du côté intérieur d'une cavité (15) en forme de sphère creuse pour réception de la tête (5), de préférence conique, de l'élément (40) d'ancrage dans l'os.

35. Dispositif selon l'une des revendications 1 à 34, **caractérisé en ce que** les mâchoires de serrage (7) présentent au moins quatre fentes parmi lesquelles un certain nombre débouche dans la surface frontale située à l'extrémité inférieure (13).

36. Dispositif selon l'une des revendications 1 à 34, **caractérisé en ce que** les mâchoires de serrage (7) présentent quatre fentes dont un certain nombre débouche dans la surface frontale située à l'extrémité supérieure (8).

37. Dispositif selon l'une des revendications 1 à 34, **caractérisé en ce que** les mâchoires de serrage (7) présentent au moins quatre fentes qui débouchent en alternance dans la surface frontale de l'extrémité inférieure (13) et dans la surface frontale de l'extrémité supérieure (8).

38. Dispositif selon l'une des revendications 1 à 23, 25 à 27 et 32 à 37, **caractérisé en ce qu'**une bague d'accouplement (22) empêche à l'extrémité supérieure (28) de la douille (10) l'évasement de la douille (10).

39. Dispositif selon la revendication 38, **caractérisé en ce que** la bague d'accouplement (22) peut être fixée sur la douille (10) par une garniture filetée (21).

40. Dispositif selon l'une des revendications 1 à 39, **caractérisé en ce que**
A) le canal (11) ménagé dans la douille (10) est ouvert en direction de l'extrémité supérieure (28);
B) le filet (19; 41) prévu à l'extrémité supérieure (28) de la douille (10) et le filet complémentaire du moyen de serrage (50) correspondant sont configurés en dents de scie, les côtés plats du profilé étant orientés de telle sorte que lors du serrage du moyen de serrage (50), la douille (10) ne s'évase pas; et
C) le côté plat du profilé forme avec l'axe central (4) un angle compris entre 87° et 93°.

41. Dispositif selon l'une des revendications 1 à 22 et 33 à 37, **caractérisé en ce que** dans la pièce de liaison (30), un canal (52) décalé transversalement par rapport à l'axe central (4) et à côté de l'alésage (6) sert à recevoir une vis (51) de fixation du support longitudinal (1).

42. Dispositif selon l'une des revendications 1 à 41, **caractérisé en ce que** la cavité (15) ménagée dans les mâchoires de serrage (7) et/ou la tête (5) de l'élément (40) d'ancrage dans l'os sont dotés d'une structuration qui présente de préférence la forme de cannelures transversales et de préférence de nervures transversales.

43. Dispositif selon l'une des revendications 1 à 42, **caractérisé en ce que** les mâchoires de serrage (7) et la tête (5) de l'élément (40) d'ancrage dans l'os sont constituées au moins dans la région de leur contact de matériaux qui présentent des duretés différentes.

44. Dispositif selon la revendication 43, **caractérisé en ce que** le matériau plus dur est de préférence un alliage de titane-aluminium-niobium et le matériau déformable est de préférence du titane pur malléable.

45. Dispositif selon l'une des revendications 1 à 42, **caractérisé en ce que** les mâchoires de serrage (7) et la tête (5) de l'élément (40) d'ancrage dans l'os présentent des duretés différentes au moins dans la région de leur contact, cette différence de dureté étant obtenue par différentes déformations à froid ou cristallisations différentes du même matériau, le matériau dur étant de préférence de l'acier 1.4441 fortement écroui et le matériau malléable de l'acier 1.4441 traité à chaud.

46. Dispositif selon l'une des revendications 1 à 42, **caractérisé en ce que** les mâchoires de serrage (7) et la tête (5) de l'élément (40) d'ancrage dans l'os présentent au moins dans la région de leur contact des duretés différentes, ces différences de dureté étant obtenues par traitement de surface, par exemple revêtement ou implantation d'ions.

47. Dispositif selon l'une des revendications 1 à 46, **caractérisé en ce que** les mâchoires de serrage (7) sont bloquées axialement en direction de l'extrémité inférieure (29) par un épaulement (31) qui s'étend perpendiculairement à l'axe central (4) et ménagé dans l'alésage (6).

48. Dispositif selon l'une des revendications 1 à 47, **caractérisé en ce que** les mâchoires de serrage (7) s'appuient en position axialement bloquée dans l'alésage (6).
